(19) Europäisches Patentamt
European Patent Office
Office européen des brevets

(11) EP 1 537 858 A1

(12) EUROPEAN PATENT APPLICATION

(43) Date of publication:
08.06.2005 Bulletin 2005/23

(51) Int Cl.7: A61K 9/127, A61K 47/00,
A61K 38/00, A61K 49/22

(21) Application number: 03027944.2

(22) Date of filing: 04.12.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(71) Applicant: Vectron Therapeutics AG
35037 Marburg (DE)

(72) Inventors:
• Müller, Rolf, Prof.
35037 Marburg (DE)
• Müller-Brüsselbach, Sabine, Dr.
35037 Marburg (DE)

• Hilka, Tanja
35096 Weimar-Niederwalgern (DE)
• Nahde, Thomas
35039 Marburg (DE)
• Graser, Andreas
35041 Marburg (DE)
• Höllig, Peter
63607 Wächtersberg (DE)

(74) Representative: Zwicker, Jörk
Forrester & Boehmert
Pettenkoferstrasse 20-22
80336 München (DE)

(54) **Drug delivery vehicles and uses thereof**

(57) The present invention relates to novel drug delivery vehicles their production and use.

## Fig. 9

**Description**

The present invention relates to novel drug delivery vehicles comprising cholesterol and sphingomyelin, methods for making and using them, in particular for the treatment of proliferative diseases, immune diseases, infectious diseases, vascular diseases, rheumatoid diseases and inflammatory diseases.

Currently most drugs are administered to the patient in a free form which means that they are in solution and not attached or incorporated into a vehicle. The term "free form" also comprises chemical derivatives of a given drug as well as various addition salts that can be formed with the drug. However, it has been realized that the attachment of a drug to or the incorporation of a drug into a delivery vehicle can offer advantages if compared to the administration of the drug in its free form. Several factors, which influence the overall efficacy of a given drug can be advantageously affected by incorporation into or attachment to such a vehicle. These factors include tissue specific distribution, in particular preferential accumulation in a certain tissue of interest or at a disease site, targeting of the drug to a particular cell type, decrease of interaction with blood components and increase in circulation time. When comparing the suitability of different drug delivery vehicles the above factors are important but among such delivery vehicles also factors such as the release characteristic of the drug from the vehicle become important for their efficacy. While all of these factors will contribute to some degree to a potential improvement of the efficacy of a given drug, when attached to or incorporated into such a drug delivery vehicle, the ultimate test for a novel delivery vehicle is its efficacy in a disease animal model or in a patient when compared to the drug in its "free form" or to another vehicle.

One type of drug delivery vehicle, which has gained attention in recent years are liposomal formulations. The term "liposomes" generally refers to uni- or multilamellar lipid structures enclosing an aqueous interior, depending on the number of lipid membranes formed. Typically liposomes can be loaded with drugs, i.e. the drug is encapsulated in the interior of the liposome, and/or drugs can be attached to the liposome or incorporated into the lipid bilayer. Such drug comprising liposomal formulations have been shown to have an increased efficacy in comparison to the free drug. For example, it has been shown that a liposomal formulation including the vinca alkaloid vincristine has a greater efficacy against leukemia cells, if compared to free vincristine and that it shows a reduced overall toxicity (Mayer et al. (1993) Cancer Chemo. Pharmacol. 33: 17-24). Since liposomes can be formed of almost any lipid a large variety of different liposomal formulations are known in the art. However, very little is known about the influence that individual lipids will have on the efficacy of a drug for a given disease let alone which molar ratios of two or more different lipids will lead to lipid compositions with an improved efficacy.

EP 0 555 333 B1, for example, describes liposomes with a high cholesterol content. The liposomes comprise cholesterol and phospholipids in a cholesterol: phospholipid ratio of 0.8 to 1.2. It is taught that these vesicles are suitable for the delivery of antigens to elicit an antigenic response, i.e. the use as a vaccine is disclosed.

US 5,543,152, US 5,471,516, and US 5,814,335 teach the use of liposomal formulations comprising sphingomyelin (SM) and cholesterol (CH) and in particular liposomes comprising between 30 and 75 mol% sphingomyelin and between 25 and 50 mol% cholesterol, which are called sphingosomes, for the delivery of drugs. For these liposomes it is taught that they possess an increased acid stability, if compared to liposomes comprising, for example, distearoyl-phosphatidylcholine (DSPC) instead of SM. This increased stability towards acids is an advantage, if the respective drug is loaded into the liposome by a pH gradient-dependent encapsulation procedure as described, for example, by Mayer et al., Biochem. Biophys. Acta 1025: 143 - 151 (1990), which involves acidifying the interior of the liposome to pHs around 4. Also disclosed is an increased circulation time for vincristine formulated with sphingosomes (SM/CH 55/45 mol%) and an improved efficacy in a mouse tumor model, if compared to vincristine formulated with DSPC.

It was now surprisingly found by the present inventors, that using a significant lower SM concentration than disclosed in the prior art and a high CH concentration it was possible to generate liposomes with an increased efficacy in the treatment of diseases, in particular tumors.

The present invention, therefore, provides a liposome, wherein CH and SM are present in relation to the total molar lipid composition of the liposome at a molar ratio of about 30 to about 60 mol% and about 5 to about 20 mol%, respectively. It has been found that a concentration of above 60 mol% limits the formation of regular lipid bilayer structures and, therefore, a content of 60% CH is the upper limit for the liposomes of the present invention. On the other hand lowering the cholesterol concentration below 30 mol% appears to increase the rate of elimination of the liposomes form the circulation and, thus, decreases the biological half life of a drug, comprised in the liposome. Concomitantly the efficacy of the liposomes in therapeutic applications, in particular in tumor therapy decreases.

Similarly, an increase of SM over 20 mol% to, for example, 30 mol% reduces the efficacy of doxorubicin in an animal tumor model, while reduction of SM below 5% or complete omission of SM dramatically decreases the circulation time of encapsulated doxorubicin and in turn reduces efficacy. Thus, the present invention was made in part through a thorough analysis of the influence that the variation of different constituents of a liposome has on the efficacy of a drug and the surprising discovery that by substantial lowering the SM content, if compared to prior art liposomes, in a liposome with a high cholesterol content a liposome is obtained that exhibits a stable liposomal structure with an improved drug delivery and therapeutic efficacy.

In a preferred embodiment SM is present in relation to the total molar lipid composition of the liposome at a molar ratio of about 8 to about 19 mol%, more preferably about 10 to about 18 mol%, even more preferably about 12 to about 16 mol% and most preferably about 13 to about 15 mol%.

In a further preferred embodiment CH is present in relation to the total molar lipid composition of the liposome at a molar ratio of about 35 to about 58 mol%, more preferably of about 40 to 56 mol%, even more preferably of about 45 to about 54 mol% and most preferably of about 48 to about 52 mol%.

In a particular preferred embodiment the liposome comprises in relation to the total molar lipid composition CH and SM at a molar ratio of about 35 to about 58 mol% and of about 5 to 20 mol%, preferably of about 40 to about 58 mol% and of about 8 to about 19 mol%, respectively, and more preferably of about 45 to about 54 mol% and of about 10 to about 18 mol%, respectively, and most preferably of about 48 to about 52 mol% and of about 12 to about 16 mol%, respectively.

The remainder of the lipid, i.e. the amount of lipid which is neither SM nor CH and which is needed to add up to 100 mol% can be made up of any lipid. The term "lipid" as used here and throughout the present invention refers to any substance having fatty or fat-like properties. In general a lipid comprises an extended apolar residue (X) and usually a water soluble, polar, hydrophilic residue (Y), which can be characterized by the basic formula

$$X\text{-}Y_n$$

Wherein n equals or is greater than zero. Lipids with n=0 are termed "apolar lipids", while lipids with n ≥1 a referred to as "polar lipids". Preferred lipids, which can make up the remainder of the lipids in the liposomes of the present invention are selected from the group consisting of glycerides, glycerophospholipides, glycerophosphinolipids, glycerophosphonolipids, sulfolipids, sphingolipids, phospholipids, isoprenolides, steroids, stearines, steroles and carbohydrate containing lipids.

Of these lipids the remainder of the lipids preferably comprises one or more phospholipids. Preferably the phospholipid is selected from the group consisting of phosphatidylcholine (PC), phosphatidylserine (PS), and phosphatidylethanolamine (PE). In an even more preferred embodiment the lipids of the liposome of the present invention only consist of SM, CH and phospholipids. In this case SM and/or CH can be present in their preferred and particularly preferred concentration ranges indicated above.

Surprisingly the present inventors were also able to show that a third lipid component, PE, exerts a positive effect on the efficacy of the liposomes of the present invention by further increasing the serum residence time of the liposome. Thus, in a further embodiment the liposome of the present invention comprises PE and in a preferred embodiment the PE is present in relation to the total molar lipid composition of the liposome at a molar ratio of about 5 to about 25 mol%, preferably about 10 to about 20 mol% and more preferably about 12 to about 18 mol% and most preferably about 14 to about 16 mol%. Preferably these ratios of PE are present when at the same time SM and/or CH are comprised in the liposome in its preferred and particular preferred concentration ranges indicated above and the remainder of the lipid is made up of any lipid as defined above, however, in a particularly preferred embodiment the remainder of the lipid is selected from a phospholipid.

A further component, which can be comprised in the liposome of the present invention is PC. PC in a preferred embodiment makes up between about 15 and about 40 mol% in relation to the total molar lipid composition of the liposome. More preferably the liposome of the present invention comprises between about 20 to about 35 mol% PC.

In a particular preferred embodiment the liposome consists of SM, CH, PE and PC and SM, CH and PE are present in their preferred and particular preferred concentration ranges as outlined above and the remainder of the lipid up to 100 mol% is made up of PC.

Liposomes of the present invention can have a diameter between 10 and 1000 nm. They, however, have in a preferred embodiment a diameter of between 50 and 200 nm and more preferably between 80 and 150 nm. The diameter of the liposomes can be affected, for example, by extrusion of the liposomal composition through sieves or meshes with a known pore size. This and further methods of controlling the size are well known in the art and are described, for example, in Mayhew et al. (1984) Biochim. Biophys. Acta 775:169-174 or Olson et al. (1979) Biochim. Biophys. Acta 557:9-23.

SM is a collective term for lipids sharing a similar hydrophilic head group. However, many different apolar residues can be attached to this head group. Thus, SM isolated from different natural sources varies substantially in the length and chemical structure of the attached apolar residues and usually is a mixture of different SMs. It has been observed by the present inventors that certain SMs provide a particular good efficacy, therefore, the SM employed in the liposomes of the present invention is preferably selected from the group consisting of SM derived from milk, SM derived from egg yolk, SM derived from brain, and synthetic SM. Synthetic SM has in a preferred embodiment the SM is distearoyl-SM.

Similar to SM PE is a generic term for lipids sharing the phosphatidylethanolamine head group. It has also been observed by the present inventors that certain PEs provide a better efficacy than others, if incorporated into the liposomes of the present invention, therefore, in a preferred embodiment the PE is selected from the group consisting of PE derived from egg; PE derived from heart; PE derived from liver; PE derived from plant; PE derived from bacteria; and synthetic PE, in particular 1,2-diacyl-sn-glycero-3-PE, 1-acyl-2-acyl-sn-glycero-3-PE and/or 1,2-dilauroyl-sn-glycero-3-PE (DLPE).

In a further embodiment any of the components making up the membrane of the liposomes of the present invention can be attached to a further chemical moiety. The term chemical moiety is not particular limited. However, in preferred embodiments the chemical moiety is a targeting moiety as discussed in more detail below or a stabilizing moiety. Stabilizing moieties within the meaning of this invention increase the circulation time of the liposome once it is administered. Particular preferred stabilizing moieties are ganglioside GM1, phosphatidylinositol or PEG, particular preferred PEGs have a molecular mass between about 1,000 and about 10,000 g/mol, more preferably about 5,000 g/mol.

In a preferred embodiment the chemical moieties in particular the stabilizing moieties are attached to only a fraction of the molecules making up the membrane of the liposomes. It is preferred that between about 1 to about 20 mol% of the components of the liposomal membrane carry an attached chemical moiety, more preferably between about 3 and about 10 mol% and even more preferably about 5 mol%.

A preferred liposomal component for attachment of the chemical moiety, in particular for the stabilizing moiety is a lipid component. While different chemical moieties can be attached to different lipid components it is preferred that the chemical moiety(ies) is(are) attached to one or more of the phospholipids comprised within the liposome of the present invention. In a further preferred embodiment the one or more chemical moiety is attached to PE. In particular, if a stabilizing agent like, for example, PEG is used PE is used for attachment.

In addition to the attachment of stabilizing moieties detergents, proteins and peptides can be incorporated into the liposome for stabilizing the lipid bilayers of the liposomes of the present invention. Detergents which can be used as bilayer stabilizing components include, but are not limited to, Triton X-100, deoxycholate, octylglucoside and lyso-phosphatidylcholine. Proteins which can be used as bilayer stabilizing components include, but are not limited to, glycophorin and cytochrome oxidase. In preferred embodiments a liposome can comprise between 0.05 and 15 mol% of a stabilizing agent.

The liposomes of the present invention do not appear to exhibit a particular preference for binding to certain tumor cells. In some applications like, for example, in tumor therapy in which it is desirable that a cytotoxic drug is preferentially delivered to tumor cells a means of targeting of the liposome of the present invention, which allows targeting of the liposomes primarily to a specific site with in the body can decrease the systemic toxicity of the liposomal drug formulation or alternatively allows to administer higher amounts of a drug at the same level of toxicity. Therefore, in a further embodiment of the liposomes of the present invention a targeting moiety is attached to the liposome. As outlined above with respect to the chemical moiety in principal the targeting moiety can be attached to any component of the liposome. Preferably, the targeting moiety is: a) attached to one of the lipid components of the liposome, b) attached to a membrane protein which can be incorporated into the membrane of the liposomes of the present invention or c) is itself capable of insertion or integration in the lipid layer.

The term "attached" as used throughout this description refers to a direct or indirect, covalent or non-covalent bond and connection, respectively, between a chemical moiety in particular a targeting moiety and another component of the liposome. A wide variety of chemical groups which allow attachment as defined above are known in the art including, for example, biotin-streptavidin, amino-reactive groups (e.g. carbodiimides, hydroxylmethylphosphine, imidoester, N-hydroxysuccinimide esters, isothiocyanates, isocyanates), sulfhydryl-reactive groups (e.g. maleimides, haloacetyls, pyridyl disulfides, aziridines) carboxyl-reactive molecules (e.g. carbodiimides, carbodiimidazole, diaoalkanes), hydroxyl-reactive groups (e.g. carbonyldiimidazole, alkyl halogens, isocyanates), and can readily be selected by someone of skill in the art as appropriate.

In a preferred embodiment the targeting moiety is selected from the group consisting of a peptide or protein, in particular an antibody or fragment thereof, a single-chain antibody or fragment thereof, a receptor ligand or fragment thereof; a carbohydrate; and a ligand.

More specifically the targeting moiety can be selected from the group consisting of natural or synthetic receptor-binding peptides, in particular integrin-binding peptides such as RGD-comprising peptides; growth factors, in particular VEGF, EGF, PDGF, TGF$\alpha$, TGF$\beta$, KGF, SDGF, FGF, IGF, HGF, NGF, BDNF, neurotrophine, BMF, bombesin, M-CSF, GM-CSF, thrombopoietin, erythropoietin, SCF, SDGF, oncostatin, PDEGF, endothelin; cytokines, in particular IL-1, IL-2, IL-3, IL-4, IL-5, IL-6, IL-7, IL-8, IL-9, IL-10, IL-12, IL-13, IL-14, IL-15, interferon $\alpha$, $\beta$ or $\gamma$, tumor necrosis factors such as TNF$\alpha$, TNF$\beta$; chemokines, in particular RANTES, MCAF, MIP-1$\alpha$ or $\beta$, NAP, $\beta$-thromboglobulin; peptide hormones such as SRH, SIH, STH, MRH, MSH, PRH, PIH, prolactin, LH-RH, FSH-RH, LH/ICSH, FSH, TRH, TSH, CRH, ACTH, agiotensin, kinine, histamine; steroid hormones, in particular estrogene, gestagene, androgene, glucocorticoide; mineral corticoids; or homologous or analogous thereof; vitamins, in particular folic acid; adhesion molecules, in particular lewis X, S-lewis X, LFA-1, MAC-1, VLA-4, PECAM, vitronectin, GMP-140, ICAM-1, VCAM-1, fibronectin, laminin, B7, CD28, CD40, CD40L and selectins; viral coatproteins; monosaccharides, in particular glucose, mannose; and oligosaccharides, in particular Man2, Man3, Man4, Man5, Man6, Man7, Man8, or Man9, lewis Y, sialyl lewis Y, and lectines.

In a preferred embodiment the targeting moiety is attached to a spacer. The term "spacer" as used throughout the description refers to a chemical moiety, which serves the purpose of providing better accessibility of the targeting moiety even when it is attached to a component of the liposome, e.g. a lipid of the present invention which might otherwise sterically hinder the binding of the targeting moiety to its respective target structure. Spacers within this meaning have a linear extension of at least 0.5 nm preferably the spacer has a linear extension of between 1 and 10 nm and even more preferably between 2 and 5 nm. The spacer is preferably a linear or branched saturated or unsaturated carbohydrate chain. The carbohydrate chain preferably comprises multimeric repeats of a monomeric building block. Depending on the length of the respective monomeric building block between 2 and 10 multimeric repeats of the monomeric building blocks are preferred. In preferred embodiments the spacer is hydrophilic. The spacer can comprise a functional group which allows attachment to the targeting moiety on one terminus and another functional group on the other terminus, which allows attachment of the spacer to a component of the liposome, e.g. a lipid of the present invention.

Preferred spacers are bifunctional molecules, in particular, bifunctional polyethylene or polypropylene glycol derivatives comprising preferably between about 1 and 40 repeat units, oligopeptides comprising natural and/or synthetic amino acids. The oligopeptides preferably comprise between 1 and 40, preferably between 2 and 20 and more preferably between 2 and 10 amino acids. A particular preferred building block of a spacer is 8-amino-3, 6-dioxatanoic acid (doo) and spacers comprising between 1 to 10 repeat units of doo are preferred. Spacers comprising between 2 and 5 doo units are even more preferred and spacers comprising 3 doo units are most preferred. In the context of liposomes it has been discovered by the present inventors that there is an optimal length of the spacer, which is between 2 and 5 nm. On one hand spacers with a length of less than 0.5 nm will in most cases not provide enough distance from the liposomal surface to which the targeting moiety has been attached to allow efficient interaction, i. e. binding, between the targeting moiety and its respective target like, for example, a tumor cell. On the other hand spacers, which are longer than 10 nm show an increasing "floppiness", which is also detrimental to the interaction between the targeting moiety and its target. Thus, in a preferred embodiment the spacer has a length of between 1 and 10 nm, preferably between 2.5 and 5 nm.

In preferred embodiments of liposomes of the present invention the targeting moiety is attached to a lipid, preferably a phospholipid like, for example PE, PC or PS and preferably the lipid, which is used for attachment of a targeting moiety is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoyl-glycerosuccinyl-lysine, alpha-methoxy-omega-(1,2-dioctadecenoyloxy glyceryl) (DO), alpa-methoxy-omega-(1,2-ditetradecenoyloxy glyceryl) (DT).

As outlined above the main components and in many embodiments the only components making up the membrane of the liposome of the present invention are lipids. However, in some aspects of the present invention the membrane of the liposomes can further comprise components, which are capable of insertion/integration into the lipid layer. Examples of such components are proteins with a hydrophilic portion, including one or more membrane spanning domains or GPI-anchor, or other amphipathic molecules such as lipopeptides and glycolipids or molecules conjugated or fused to one or more fatty acid, lipid or other hydrophobic moieties. Such molecules can, for example, provide the liposome with a targeting capacity, i.e. can be a targeting moiety, or can have an enzymatic function.

As outlined above the liposomes of the present invention are superior vehicles for drugs that were shown to provide a prolonged presence of the drug in the circulation if compared to the free drug, a good release characteristics, i.e. a release of the drug from the liposome into the circulation, a low binding to blood components and a good stability against conditions of high or low pH, which are encountered, for example, during production of the liposomes. In this respect it is interesting to note that the stability in the circulation of prior art liposomal drug formulations like, for example, Doxil® (a trademark of Ortho Biotech Products, L.P.), which has a 50% value, i.e. the time point at which only 50% of the initially administered drug detectable in the circulation, in excess of 6 hours, while the AVE 95-dox formulation of the present invention only had 50% value of about 30 min, has a much lower efficacy than AVE 95-dox in a mouse tumor model as judged by the growth inhibition of the tumors. Since the liposomes of the present invention are superior delivery vehicles the liposomes of the present invention comprise in a preferred aspect at least one drug and/or at least one diagnostic. A drug within the meaning of the present invention is any compound, which exerts a therapeutic effect upon administration.

It is particularly preferred that the drug or diagnostic is comprised in the interior of the liposome or in cases of lipophilic drugs also within or between the lipid bilayers. A variety of methods are available in the prior art to "load" a liposome with a given drug or diagnostic. In its simplest form the drug and/or diagnostic is/are admixed with the lipid components during formation of the liposomes. Other passive loading methods include dehydration-rehydration (Kirby & Gregoriadis (1984) Biotechnology 2:979), revers-phase evaporation (Szoka & Papahadjopoulos (1978) Proc. Natl.Acad. Sci. USA 75: 4194-), or detergent-depeletion (Milsmann et al. (1978) Biochim. Biophys. Acta 512:147-155). However, these techniques often lead to a substantial loss of drug during loading, which is a particular disadvantage in cases where the drug is expensive.

Other methodologies for encapsulating drugs and/or diagnostics include so called "remote loading" or "active loading" in which due to a gradient, for example, a pH or salt gradient between the exterior and the interior of a preformed liposome the drug and/or diagnostic is transported into the liposome along the gradient (see, for example Cheung et al. (1998) Biochim. Biophys. Acta 1414:205-216; Cullis et al. (1991) Trends Biotechnol. 9:268-272; Mayer et al. (1986) Chem. Phys. Lipids 40: 333-345).

The passive and active loading techniques referred to above and other methods well known in the art can all without limitation employed by the skilled artisan. The most efficient method of loading for any given drug or diagnostic can be determined by routine experimentations by well established procedures. Variables which are typically adjusted are pH, temperature, salt type and concentration, type of buffer etc.

In a preferred embodiment the drugs and/or diagnostics are loaded by remote loading into the liposomes, since this method offers a very low loss of the substance to be loaded. In a preferred embodiment a pH gradient is used for loading. Depending on the substance to be loaded the interior of the liposome will typically be acidified with respect to its exterior. Preferably the interior will have a pH between 1 and 6 prior to loading with the drug or diagnostic.

In a preferred embodiment the drug is selected from the group consisting of analgesics, antirheumatics, anthelminthics, antiallergics, antianemics, antiarrhythmics, antibiotics, angiogenesis inhibitors, antiinfectives, antidemenics (nootropics), antidiabetics, antidotes, antiemetics, antivertiginosics, antiepileptics, antihemorrhagics, antihypertonics, antihypotonics, anticoagulants, antimycotics, antitussiv agents, antiviral agents, beta-receptor and calcium channel antagonists, broncholytic and antiastmatic agent, chemokines, cytokines, mitogens, cytostatics, cytotoxic agents and prodrugs thereof, dermatics, hypnotics and sedatives, immunosuppressants, immunostimulants, peptide or protein drugs, in particular hormones and physiological or pharmacological inhibitors of mitogens, chemokines, or cytokines or their respective prodrugs. Of course it is also envisioned that a liposome of the invention comprises more than one drug at once.

As previously outlined due to its circulation time and release characteristics the liposomes of the present invention are particularly suited for the therapy of proliferative diseases in which an inhibition of the cellular proliferation needs to be achieved, therefore, the liposome can comprise any cytostatic or cytotoxic drug, however, from the known cytostatics and cytotoxic drugs the following are particularly preferred: alkylating substances, anti-metabolites, antibiotics, epothilones, nuclear receptor agonists and antagonists, anti-androgenes, anti-estrogens, platinum compounds, hormones and antihormones, interferons and inhibitors of cell cycle-dependent protein kinases (CDKs), inhibitors of cyclooxygenases and/or lipoxygenases, biogeneic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof. Several of the above indicated drugs are now administered simultaneously for cancer therapy and, consequently, it is also envisioned that more than one cytostatic and/or cytotoxic drug is comprised in a liposome of the present invention.

In another aspect the liposomes of the present invention can comprise anti-angiogenic drugs like, for example, fumagillin analogs, thalidomide, 2-methoxyestradiol, protein tyrosine kinase inhibitors. Such drugs are preferred for the treatment of diseases involving activated and/or proliferating endothelial cells For the treatment of diseases involving activated and/or proliferating endothelial cells, however, it is also possible to use cytostatic or cytotoxic drugs in particular those, which are indicated as preferred cytostatic or cytotoxic drugs above.

The term "immunosuppressant" comprises both substances which lower the activity of immune response as well as substances with an anti-inflammatory action, preferred examples are glucocorticoids, in particular beclomethasone, betamethasone, clocortolone, cloprednol, cortisone, dexamethasone, fludrocortisone, fludroxycortide, flumetasone, fluocinolone acetonide, fluocinonide, fluocortolone, fluorometholone, fluprednidene acetate, hydrocortisone, paramethasone, prednisolone, prednisone, prednylidene, pregnenolone, triamcinolone or triamcinolone acetonide, a cyclosporin, in particular cyclosporin A, mycophenolate mofetil, tacrolimus, rapamycin, FK 506, cycloheximide-N-(ethyl ethanoate), azathioprine, ganciclovir, an anti-lymphocyte globulin, ascomycin, myriocin, a pharmacological inhibitor of MAP kinases (especially a p38 inhibitor such as VX-745), caspase inhibitors, matrix metalloproteinase inhibitors, and/or methotrexate.

The term "immunostimulant" encompasses all substances, which influence the function of cells which are involved directly or indirectly in mediation of the immune response, and where the influence leads to an immune response. These cells include, for example, macrophages, Langerhans cells and other dendritic cells, lymphocytes, indeterminate cells, but also cells which do not themselves belong to the immune system but are involved in immune disorders of the skin, such as fibroblasts, keratinocytes and melanocytes, but especially Langerhans cells. The strength of the immune response can be determined for example through the amount of cytokines produced (such as interferon-gamma), detection of activation markers on dendritic cells (such as MHCII or CD86) or the number of activated CD8-positive T cells in the skin. Immunostimulants for the purpose of the present invention are, in particular, plant immunostimulants which are obtained, for example, from Echinacea pallida or Echinacea purpurea, cytokines such as, for example, interleukins, interferons and colony-stimulating factors, and bacterial constituents or molecules which mimic the latter [such as bacterial DNA and unmethylated oligodeoxynucleotides with CpG sequences, and constituents of the bacterial cell wall or coat, especially the lipopolysaccharides and molecules derived therefrom, such as monophosphoryl-lipid A, muramyldipeptide (N-acetylmuramyl-L-alanyl-D-isoglutamine), and/or PamCys3, and other molecules such as tetanus toxoid, poly-L-arginine or MHCII peptides].

The term "antibiotics" encompasses, for example, penicillins, cephalosporins, tetracyclines, aminoglycosides, macrolide antibiotics, lincosamides, gyrase inhibitors, sulfonamides, trimethoprim, polypeptide antibiotics, nitroimidazole derivatives, amphenicol, in particular actinomycin, alamethicin, alexidine, 6-aminopenicillanic acid, amoxicillin, amphotericin, ampicillin, anisomycin, antiamoebin, antimycin, aphidicolin, azidamfenicol, azidocillin, bacitracin, beclomethasone, benzathine, benzylpenicillin, bleomycin, bleomycin sulfate, calcium ionophore A23187, capreomycin, carbenicillin, cefacetrile, cefaclor, cefamandole nafate, cefazolin, cefalexin, cefaloglycin, cefaloridine, cefalotin, cefapirin, cefazolin, cefoperazone, ceftriaxone, cefuroxime, cephalexin, cephaloglycin, cephalothin, cephapirin, cerulenin, chloramphenicol, chlortetracycline, chloramphenicol diacetate, ciclacillin, clindamycin, chlormadinone acetate, chlorpheniramine, chromomycin A3, cinnarizine, ciprofloxacin, clotrimazole, cloxacillin, colistine methanesulfonate, cycloserine, deacetylanisomycin, demeclocycline, 4,4'-diaminodiphenyl sulfone, diaveridine, dicloxacillin, dihydrostreptomycin, dipyridamole, doxorubicin, doxycycline, epicillin, erythromycin, erythromycin stolate, erythromycin ethyl succinate, erythromycin stearate, ethambutol, flucloxacillin, fluocinolone acetonide, 5-fluorocytosine, filipin, formycin, fumaramidomycin, furaltadone, fusidic acid, geneticin, gentamycin, gentamycin sulfate, gliotoxin, gramicidin, griseofulvin, helvolic acid, hemolysin, hetacillin, kasugamycin, kanamycin (A), lasalocid, lincomycin, magnesidin, melphalan, metacycline, meticillin, mevinolin, micamycin, mithramycin, mithramycin A, mithramycin complex, mitomycin, minocycline, mycophenolic acid, myxothiazole, natamycin, nafcillin, neomycin, neomycin sulfate, 5-nitro-2-furaldehyde semicarbazone, novobiocin, nystatin, oleandomycin, oleandomycin phosphate, oxacihin, oxytetracycline, paromomycin, penicillin, pecilocin, pheneticillin, phenoxymethylpenicillin, phenyl aminosalicylate, phleomycin, pivampicillin, polymyxin B, propicillin, puromycin, puromycin aminonucleoside, puromycin aminonucleoside 5'-monophosphate, pyridinol carbamate, rolitetracycline, rifampicin, rifamycin B, rifamycin SV, spectinomycin, spiramycin, streptomycin, streptomycin sulfate, sulfabenzamide, sulfadimethoxine, sulfamethizole, sulfamethoxazole, tetracycline, thiamphenicol, tobramycin, troleandomycin, tunicamycin, tunicamycin A1 homolog, tunicamycin A2 homolog, valinomycin, vancomycin, vinomycin A1, virginiamycin M1, viomycin and/or xylostasin.

The term "antiinfectives" encompasses, for example, antimycotics, agents with antiparasitic effect and virustatics, in particular amphotericin, vifonazole, buclosamide, quinoline sulfate, chlormidazole, chlorphenesin, chlorquinaldol, clodantoin, cloxiquine, cyclopirox olamine, dequalinium chloride, dimazole, fenticlor, flucytosine, griseofulvin, ketoconazole, miconazole, natamycin, sulbentine, tioconazole, toinaftate, antiretroviral agents and/or herpes remedies.

The term "antiallergics" encompasses, for example, substances from the class of globulins, corticoids or antihistamines, in particular beclomethasone and derivatives thereof, betamethasone cortisone and derivatives thereof, dexamethasone and derivatives thereof, bamipine acetate, buclizine, clemastine, clemizole, cromoglicic acid, cyproheptadine, diflucortolone valerate, dimetotiazine, diphenhydramine, diphenylpyraline, ephedrine, fluocinolone, histapyrrodine, isothipendyl, methdilazine, oxomemazine, paramethasone, prednylidene, theophylline, and/or tolpropamine tritoqualine.

The term "mitogens", "chemokines" and "cytokines" encompass, for example, interferon-alpha, interferon-beta, interferon-gamma, interleukin-1, interleukin-2, interleukin-7, interleukin-10, interleukin-12, interleukin-18, GM-CSF, MIP-1-alpha/beta, RANTES, EGF, basic or acidic FGF, PDGF, IGF, VEGF, TGF-beta and/or TNF-alpha.

The term "dermatics" encompasses, for example, shale oil sulfonates, tar and tar derivatives, astringents, antihidrotics, acne remedies, antpsoriatics, antiseborrheic agents and/or enzyme preparations for the treatment of skin defects.

As the liposomes of the present invention show an advantageous release pattern thy can also be used to deliver a diagnostic to a certain tissue. This is particular preferred in the context of a targeting moiety, which preferentially localizes the liposomes of the present invention in certain tissues or disease sites. In preferred embodiments the diagnostic is selected from the group consisting of an electron dense molecule, a paramagnetic molecule, a superparamagnetic molecule, a radioactive molecule, a non-radioaktive isotope, and a fluorescent molecule.

The term "marker" refers to a chemical moiety which is directly or indirectly detectable by analytical methods including measurement of fluorescence, nuclear magnetic resonance, computer tomography or scintigrams and comprises without limitation electron dense molecules, paramagnetic molecules, superparamagnetic molecules, radioactive molecules like, for example, $^{13}$N, $^{15}$O, $^{18}$F, $^{51}$Gr, $^{54}$Fe, $^{60}$Co, $^{67}$Ga, $^{75}$Se, $^{99m}$Tc, $^{111}$In, $^{112m}$Ag, $^{113m}$In, $^{123}$I, $^{133}$Xe, $^{148}$Au, $^{35}$S, $^{33}$P, $^{32}$P, or $^{11}$C, non-radioactive isotopes, which include, for example, $^{2}$H and $^{13}$C, and fluorescent molecules or molecules generating fluorescence or light emission like, for example, green fluorescent protein, luciferase, and a variety of fluorescent dies all of which are well known to someone of skill in the art.

The liposome can comprise a variety of additional substances preferably in the membrane or in the interior of stabilizers, protectants, metal ion chelators, buffers and/or additives are comprised in the liposome.

The liposomes of the present invention are stable structures, which can, for example, be filtered after production to remove surrounding drug or buffer. The pure liposomes with or without drugs and/or diagnostics can be used, however, due to its stability it is also possible to remove any liquid from the liposome to facilitate easy storage. Therefore, the liposomes of the present invention can be supplied in dried form, preferably in a freeze dried form. These liposomes can than be readily rehydrated upon addition of water or buffer at the time of use.

A further aspect of the present invention is a method for producing a liposome of the present invention, in which SM, CH and remaining lipid(s) or other components are mixed. Preferably the remaining lipid is selected from PE and/or PC. If no drugs or diagnostics are mixed initially with the lipids drugs and/or diagnostics are then in a second step loaded into the liposomes, Since "remote loading" is a preferred way of introducing a substance preferably a drug and/or diagnostic into the liposome the method of the present invention can in a preferred embodiment involve a remote loading step, preferably employing a pH gradient.

A further aspect of the present invention is a pharmaceutical composition comprising a liposome of the present invention or a liposome produced according to the method of the present invention, further comprising stabilizers, protectants, metal ion chelators, buffers and/or additives.

A further aspect is a diagnostic composition comprising a liposome of the present invention or a liposome produced according to the method of the present invention, further comprising stabilizers, protectants, metal ion chelators, buffers and/or additives.

The liposomes of the present invention, the pharmaceutical composition of the present invention or the diagnostic composition of the present invention preferably comprise, stabilizers which are selected from the group consisting of α-tocopherol, vitamin E or carbohydrates, in particular glucose, sorbitol, sucrose, maltose, trehalose, lactose, cellubiose, raffinose, maltotriose, or dextran.

The liposomes and liposomal compositions disclosed herein are particular capable delivery vehicles or a pharmaceutical composition of the present invention for the production of a medicament for the therapy of proliferative diseases, immune diseases, in particular autoimmune disease, infectious diseases, vascular diseases, rheumatoid diseases, in particular osteoarthritis or rheumatoid arthritis, inflammatory diseases or any disease or condition, which is associated with the damage or a permeability increase of the vasculature, including but not limited to mechanic injury stroke or hemorrhages. The drugs and/or diagnostics comprised in the liposomes of the present invention can be administered to patients in amounts similar or identical to the amounts in which the free drugs or diagnostics are typically administered. Suitable doses and administration schemes are known from a variety of sources including, for example, Berger et al. "Das Rote Buch" (1997) Publisher Ecomed Landsberg Lech. For example, doxorubicin is typically administered in a range from 45 to 75 mg/m$^2$ per day every 3 to 4 weeks or 10 to 20 mg/m$^2$ weekly. However, it is also within the capabilities of the attending physician to increase or decrease the dose or administration frequency of the respective drug comprised in the liposome based on the level of toxicity observed.

The liposomes or pharmaceutical compositions can be administered through a variety of ways including intra-muscular, intravenous, intranasal, intraperitoneal, intradermal, or subcutan. The compounds can also be injected directly into the disease site.

Further experiments performed by the present inventors have shown that the liposomes and pharmaceutical compositions have a superior efficacy in the treatment of tumors and, therefore, in a preferred embodiment the proliferative disease is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

Similarly a further aspect of the invention concerns the use of a diagnostic composition of the invention for the diagnosis of a disease selected from the group of proliferative diseases, immune diseases, infectious diseases, vascular diseases, rheumatoid diseases, and inflammatory diseases.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

## Description of the Figures

**Fig. 1:** Cryoelectron microscopy of AVE9.

**Fig. 2:** Interaction of various AVE formulations with human plasma. Fraction 4 = liposome fraction, fraction 11 = main protein fraction.

**Fig. 3:** Binding of AVE9 and AVE3 to various tumor cell lines analyzed by flow cytometry using rhodamine-labeled liposomes.

**Fig. 4:** Pharmacokinetics of free doxorubicin and doxorubicin encapsulated into AVE9 or AVE95 in comparison to Doxil.

**Fig. 5:** Antitumor effects of doxorubicin encapsulated into AVE9 or AVE95 in comparison to unencapsulated doxorubicin. C26 tumor-bearing mice were injected three-times (day 1, 3, and 6) with doxorubicin at 4 mg/kg body weight.

**Fig. 6:** Comparison of antitumor effects of AVE95-dox with pegylated liposomes (Doxil). C26 tumor-bearing mice were injected three-times (day 1, 3, and 6) with doxorubicin at 4 mg/kg body weight.

**Fig. 7:** Antitumor effects of AVE95-dox and liposomes composed of 30 mol% SM, 50 mol% cholesterol and 20 mol% PC (30/50-dox) containing entrapped doxorubicin. C26 tumor-bearing mice were injected three-times (day 1, 3, and 6) with doxorubicin at 4 mg/kg body weight.

**Fig. 8:** Pharmacokinetics of free mitoxantrone and mitoxantrone encapsulated into AVE95.

**Fig. 9:** Antitumor effects of AVE95-mitoxantrone on C26 colon carcinoma tumors using a mitoxantrone dose of 4 mg/kg body weight injected at day 1, 3 and 6.

## Examples

### Example 1: AVE9 are long-circulating liposomes

We analyzed various liposomal formulations for their pharmacokinetic behavior in athymic nu/nu mice (Harlan; "nude mice"): AVE3 (33.3 mol% cholesterol, DLPE, DOPS) highly negatively charged liposomes, AVE5 (33.3 mol% cholesterol, DLPE, DOPG) highly negatively charged liposomes, AVE7 (35 mol% cholesterol, 15.4 mol% egg PC, 14.7 mol% DLPE, 16.7 mol% DOPS, 18.2 mol% milk SM), AVE9 (35 mol% cholesterol, 32,1 mol% POPC, 14,7 mol% DLPE, 18.2 mol% milk SM), AVE11 (35 mol% cholesterol, 28,8 mol% POPC, 14,7 mol% DLPE, 3,4 mol% DOPS, 18.2 mol% milk SM), AVE14 (33.3 mol% cholesterol, 33.3 mol% egg PC, 33.3 mol% DLPE).

All lipids were purchased from Avanti Polar Lipids (USA) and were used without further purification. Liposomes were prepared from dried lipid films by hydration. For this purpose lipids were dissolved in chloroform or chloroform/methanol (1:1) and mixed at the indicated ratios. For pharmacokinetic studies tritium-labeled cholesteryloleoylether (10 µCi/µmol lipid) was added. Lipids were dried using a rotary evaporator and residual solvent was removed under high vacuum. Lipid films were then hydrated with 10 mM Tris-HCl pH 7.4 to a final lipid concentration of 10 µmol/ml. Liposomes were extruded 21-times through 50 nm membranes. All liposomes prepared had an average size of 80 - 110 nm. Average zeta potentials were -67 mV for AVE3, -43 mV for AVE5, -49 mV for AVE7, -8 mV for AVE9, and -12 mV for AVE14.

Liposomes (1 µmol lipid in PBS) were injected i.v. into the tail veins of nude mice. Blood samples were taken at varying time points and analyzed for radioactivity by scintillation counting. For the analysis of tumor and organ accumulation liposomes were injected into nude mice bearing established subcutaneous murine C26 colon carcinomas. This study showed that AVE3, AVE5 and AVE7 were rapidly cleared from circulation (50% value < 5 min). Prolonged circulation times were observed for AVE9 (50% value approx. 70 - 80 min), AVE11 (50% value approx. 10 min), and AVE14 (50% value approx. 30 min). Thus, liposomes based on the AVE9 lipid composition showed a highly extended circulation in the blood stream. This was also reflected by the organ distribution determined 6 hours post-injection. While AVE3, AVE5 and AVE7 showed a high liver uptake (40-60%) and a low accumulation in the tumor (< 0.5% initial dose), especially AVE9 showed reduced liver uptake (approx. 10-20%) and an increased tumor accumulation (2-4%). These results indicate that AVE9 liposomes composed of cholesterol, phosphatidylcholine, sphingomyelin and phosphatidylethanolamine possess favorable *in vivo* kinetics.

### Example 2: Cryoelectron microscopy of empty AVE9 liposomes

A 500 mesh copper grid was coated using 5 µl of the liposomal preparation. After removing the supernatant the sample was frozen in ethane at -170°C. The grid was transferred to a liquid nitrogen cooled grid holder and examined in an electron microscope.

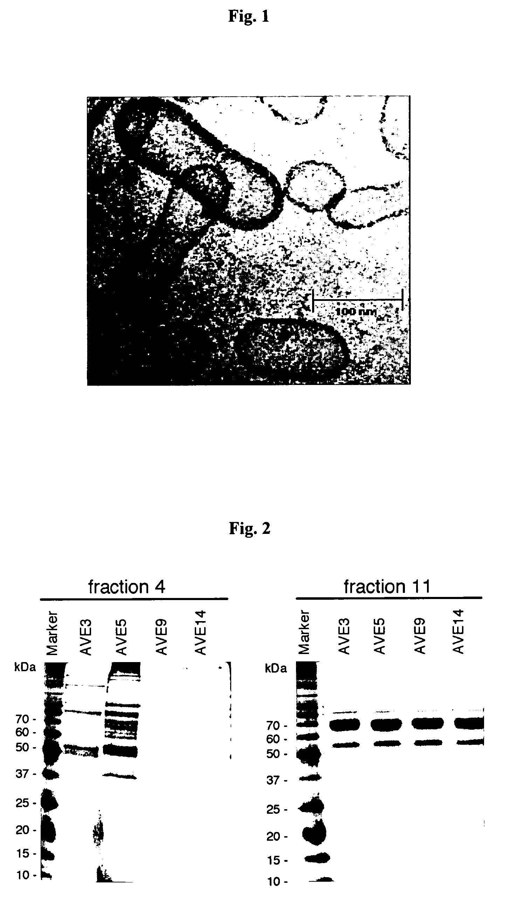

AVE9 liposomes appeared as slightly oval and mainly unilamellar structures with a size between 50 - 200 nm (Fig. 1).

### Example 3: Stability of empty AVE9 liposomes

After preparation of AVE9 liposomes as described in example 1, liposomes were stored at 4 - 8°C and particle size was analyzed weekly for up to 120 days by photon correlation spectroscopy (PCS). No increase in particle size was observed over this period of time indicating that AVE9 liposomes are stable under storage conditions.

### Example 4: AVE9 liposomes show no or only weak binding to plasma proteins

The interaction of AVE9 liposomes with plasma proteins was analyzed by incubating liposomes with human plasma at 37°C for 1 hour. Subsequently liposomes were separated from plasma proteins by size exclusion chromatography on a sepharose 4B column. In initial experiments using tritium-labeled liposomes it was demonstrated that liposomes eluted in fraction 4-5, while proteins eluted between fractions 6-20 with the main peak eluting in fractions 9-12. In these experiments we included AVE3, AVE5 and AVE14 (see example 1) for comparison. After separation of plasma-incubated liposomes by size exclusion chromatography, proteins present in fraction 4 (liposome fraction) or fraction 11 (main protein fraction) were precipitated with trichloracetic acid, equal volumes were separated by SDS-PAGE under reducing conditions and proteins were subsequently visualized by silver staining. Binding of a large number of plasma proteins was observed for highly negatively charged AVE3 and AVE5 liposomes. In contrast AVE9 but also AV14 showed no or only week binding of plasma proteins (Fig. 2). The presence of plasma proteins in the original samples was confirmed by the presence of equal amounts of protein (mainly albumin) in fraction 11. These results demonstrate that AVE9 do not or only weekly interact with plasma proteins in vitro under physiological conditions.

### Example 5: AVE9 liposomes do not interact with complement factors

The interaction of AVE9 liposomes with complement factors was analyzed using a hemolytic complement assay (plates with agarose-fixed sensitized sheep erythrocytes). AVE3 were included in these experiments as liposomes showing strong binding of plasma proteins. For this purpose liposomes at a concentration of 0,5 to 16 nmol lipid were incubated with human serum. AVE3 at a concentration between 2 to 16 nmol showed strong binding of complement factors as indicated by an inhibition of complement-mediated lysis. In contrast, AVE9 did not bind complement factors at the applied lipid concentrations further demonstrating that AVE9 show no or only weak interaction with plasma proteins.

## Example 6: Cell binding properties of AVE9 liposomes

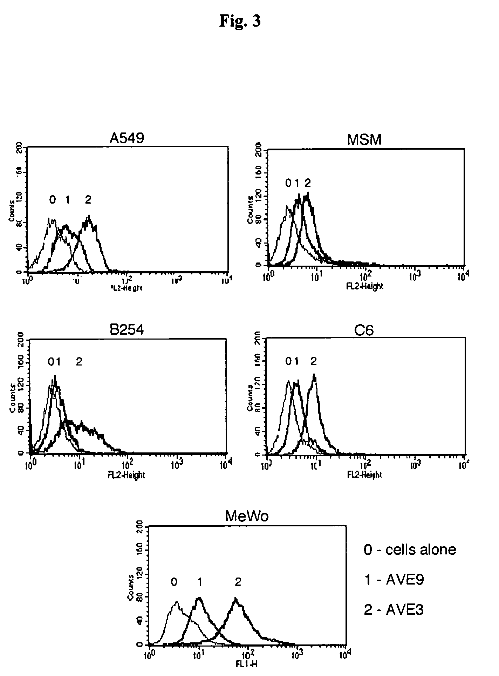

The binding properties of AVE9 liposomes to various tumor cell lines (human melanoma cell lines Me-Wo, B254 and MSM, human lung carcinoma cell line A549, rat glioblastoma cell line C6) was analyzed with rhodamine-labeled liposomes containing 0,3 mol% Rh-DPPE in the lipid bilayer. Liposomes were prepared as described in example 1. AVE3 liposomes were included to compare binding of AVE9 liposomes with that of highly negatively charged liposomes. Tumor cells were incubated with liposomes (100 nmol lipid) at 4°C for 30 min and subsequently analyzed by flow cytometry for cell binding activity. All cell lines tested showed only marginal binding of AVE9 (approximately 2 to 3-fold increase of fluorescence compared to cells incubated without liposomes), while AVE3 liposomes showed strong binding (> 10-fold increase in fluorescence) to most of the cell lines (Fig. 3).

## Example 7: Influence of cholesterol on pharmacokinetics of AVE9 liposomes

The effects of different cholesterol concentrations on pharmacokinetics were analyzed for liposomes containing between 10 to 50 mol% cholesterol. For this purpose the following liposomes based on the AVE9 formulation but with different cholesterol concentrations were prepared: AVE9 (35 mol% cholesterol, 32,1 mol% POPC, 14.7 mol% DLPE, 18.2 mol% bovine milk SM), AVE91 (10 mol% cholesterol, 44,4 mol% POPC, 20,4 mol% DLPE, 25,2 mol% bovine milk SM), AVE92 (20 mol% cholesterol, 39,5 mol% POPC, 18,1 mol% DLPE, 22,2 mol% bovine milk SM), AVE93 (30 mol% cholesterol, 34,6 mol% POPC, 15,8 mol% DLPE, 19,6 mol% bovine milk SM), AVE94 (40 mol% cholesterol, 29,6 mol% POPC, 13,6 mol% DLPE, 16,8 mol% bovine milk SM), and AVE95 (50 mol% cholesterol, 24,7 mol% POPC, 11,3 mol% DLPE, 14,0 mol% bovine milk SM). For pharmacokinetic studies tritium-labeled cholesteryloleoylether (10 µCi/µmol lipid) was incorporated into the lipid bilayer. Liposomes were prepared from dried lipid films by hydration with 10 mM Tris-HCl pH 7.4 as described in example 1. All liposomes had similar zeta potentials, which were in the range of -8 to -3 mV. Particle sizes ranged from 70 nm for AVE91 to 105 nm for AVE95, indicating that particle size slightly increased with increasing cholesterol concentrations.

Liposomes (1 µmol lipid in PBS) were injected i.v. into the tail veins of nude mice. Blood samples were taken at varying time points and analyzed for radioactivity by scintillation. Analysis of pharmacokinetic behavior demonstrated that liposomes containing 10 mol% cholesterol were much faster eliminated from circulation (50% value approx. 25 min) than formulations containing between 20 - 50% cholesterol, which had a prolonged circulation time (50% values between 70-100 min). No significant differences were observed for the organ distribution of all formulations tested.

## Example 8: Influence of spingomyelin and phosphatidylethanolamine on pharmacokinetics of AVE9 liposomes

The pharmacokinetics of AVE9 formulations containing different sphingomyelin concentrations or AVE9 formulation lacking phosphatidylethanolamine were analyzed in comparison to AVE9 (see example 1). Following formulations were tested: AVE9-0SM (35 mol% cholesterol, 50,3 mol% POPC, 14,7 mol% DLPE), AVE9-5SM (35 mol% cholesterol, 45,3 mol% POPC, 14,7 mol% DLPE, 5 mol% milk SM), AVE9-30SM (35 mol% cholesterol, 20,3 mol% POPC, 14,7 mol% DLPE, 30 mol% milk SM), AVE9-0PE (35 mol% cholesterol, 46,8 mol% POPC, 18.2 mol% milk SM). Liposomes were prepared and analyzed for pharmacokinetic properties as described in example 1.

Analysis of pharmacokinetic behavior demonstrated that liposomes containing 30 mol% SM had a reduced serum residence time (50% value = 19 min) compared to AVE9 and AVE9 containing 5 mol% SM (50% value = 70-80 min). Liposomes lacking SM (AVE9-0SM) had also a reduced serum residence time (50% value = 32 min). AVE9 lacking PE had a reduced serum residence time (50% value = 60 min). Thus, these experiments indicate that pharmacokinetics are strongly influenced by the SM concentration with an optimal SM concentration between 5 to 20 mol%.

## Example 9: Influence of pegylation on pharmacokinetics of AVE9 liposomes

The effects of pegylation on pharmacokinetics of AVE9 was analyzed for AVE9-5%PEG5000 (33,2 mol% cholesterol, 30,5 mol% POPC, 14 mol% DLPE, 17,3 mol% bovine milk SM, 5 mol% DPPE-PEG5000) and AVE9-0SM/5%PEG5000 (33,2 mol% cholesterol, 47,8 mol% POPC, 14 mol% DLPE, 5 mol% DPPE-PEG5000) in comparison to AVE9 and other formulations described in example 3. Liposomes were prepared and analyzed for pharmacokinetic properties as described in example 1.

Analysis of pharmacokinetic behavior demonstrated that pegylation of AVE9 liposomes further extended circulation time (50% value 320 min for pegylated AVE9 compared to 75 min for AVE9). Pegylated AVE9 liposomes lacking sphingomyelin (AVE9-0SM/5%PEG5000) had a reduced circulation time (50% value 43 min). These findings indicate that sphingomyelin strongly influences pharmacokinetics and that sphingomyelin can be considered a substitute for pegylation in respect to pharmacokinetic properties.

**Example 10: Encapsulation of doxorubicin into AVE9 and AVE95**

Doxorubicin was encapsulated into AVE9 or AVE95 liposomes (see examples 1 and 7). For this purpose lipid films were hydrated with 300 mM citrate buffer pH 4.0. After extrusion the pH of the external aqueous solution was adjusted to pH 7.4 with NaOH. Liposomes were heated to 60°C and doxorubicin in PBS was added and incubated for 15 min. A drug to lipid ratio of 1:5 (w/w) was routinely used in these experiments. Unencapsulated doxorubicin was removed by ultrafiltration using Vivaspin columns. Encapsulation efficiency was determined by reverse-phase HPLC analysis. By this approach approximately 70-90% of doxorubicin could be routinely encapsulated. No differences in encapsulation efficiency were observed for AVE9 and AVE95 (see example 1 and 7 for lipid compositions) at the indicated drug to lipid ratio indicating that the cholesterol concentration has no influence on encapsulation efficiency. Doxorubicin-containing AVE9 or AVE95 had a particle size which was approximately 10 nm increased compared to empty liposomes. The average size was however below 120 nm as determined by PCS. Cryo-electron microscopy revealed mainly unilamellar particles with sizes between 70-150 nm with a round or oval structure. Upon storage at 4 - 8°C particle sizes of AVE9-dox and AVE95-dox slightly increased by 10 nm over a period of 6 months. Under these storage conditions we observed only a low release (< 5%) of encapsulated drug over a period of 7 weeks.

**Example 11: Pharmacokinetics of AVE9-doxorubicin liposomes**

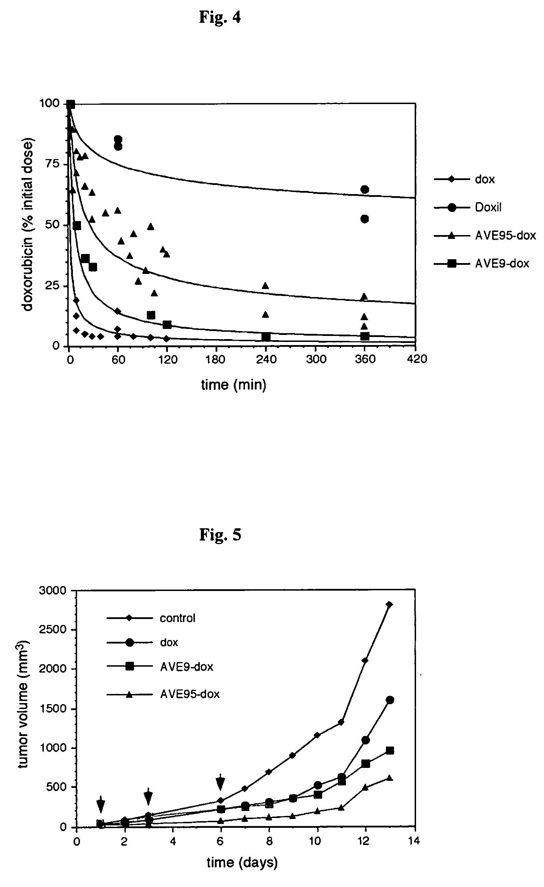

AVE9-dox and AVE95-dox were injected i.v. into the tail veins of nude mice and blood concentration of doxorubicin was determined by HPLC at varying time points. In this experiment we included unencapsulated doxorubicin as well as doxorubicin encapsulated into pegylated "stealth liposomes" (Doxil®). Compared to AVE9-dox AVE95-dox caused a prolonged serum residence time of doxorubicin (50% value of 11 min for AVE9-dox versus 23 min for AVE95-dox). Unencapsulated dox was rapidly cleared from the circulation (50% value = < 3 min). In contrast, after injection of Doxil® more than 50% of initial doxorubicin was present in the blood after 6 hours (Fig. 4). Compared to free doxorubicin AVE95-dox showed a reduced accumulation of the drug in the lung, heart and kidneys. Doxorubicin concentrations in the liver and spleen were increased 3-fold after applying AVE95-dox compared to free doxorubicin. In addition, injection of AVE95-dox and free doxorubicin into C26-tumor-bearing mice resulted in increased accumulation (approximately 2 to 3-fold after 6 hours) of doxorubicin in the tumor tissue for AVE95-dox. These data could be confirmed by fluorescence microscopy of cryo-sections of various organs and tumors.

**Example 12: Pharmacodynamics of AVE9 and AVE95-doxorubicin liposomes**

For further studies pharmacodynamic experiments in a C26 murine colon carcinoma model were performed. For this purpose tumor cells were injected subcutaneously into nude mice. After tumors had reached a size of approximately 50-100 mm$^3$ tumor-bearing mice were treated with doxorubicin-loaded liposomes injecting a doxorubicin dose of 4 mg/kg body weight at days 1, 3 and 6.

In a first experiment AVE9-dox and AVE95-dox (see example 10) were compared to unencapsulated doxorubicin. In this experiment we observed reduced tumor growth and a prolonged survival time for animals treated with AVE95-dox compared to animals treated with AVE9-dox or free doxorubicin. All animals showed reduced tumor growth compared to untreated control animals (Fig. 5). The difference between free doxorubicin and AVE95-dox at day 12 was statistically significant (p = 0.05). This was also reflected by the mean survival time which was 20 days for AVE95-dox treated animals, 16 days for AVE9-dox treated animals, 14 days for doxorubicin-treated animals, and 12 days for untreated animals.

In a second experiment free doxorubicin was compared to AVE95-dox, when administered either at 4 or at 8 mg/kg body weight to C26-tumor-bearing mice. No differences in tumor growth were observed for free doxorubicin at these two concentrations. In contrast, tumor growth was further reduced using 8 mg/kg AVE95-dox compared to 4 mg/ml AVE95-dox.

However, an increased lethality was observed for both free and encapsulated doxorubicin at 8 mg/ml.

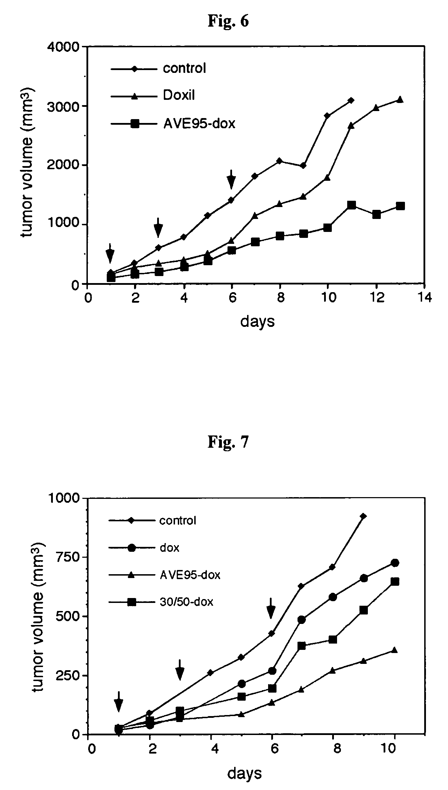

In a third experiment AVE95-dox was compared to Doxil® for the treatment of C26 tumors applying a dose of 4 mg/kg. Compared to Doxil administration of AVE95-dox resulted in a statistically reduced tumor growth (p < 0.05) and a prolonged survival time (Fig. 6).

In a final set of experiments, AVE95-dox liposomes were compared to a AVE95-based formulation containing varying sphingomyelin concentrations. Firstly, AVE95-dox was compared to the same formulation in which sphingomyelin was substituted by phosphatidylcholine (AVE95-0SM-dox). The removal of sphingomyelin drastically reduced efficacy to that seen with free doxorubicin. Secondly, AVE95-dox was compared to liposomes consisting of 50 mol% cholesterol, 30 mol% sphingomyelin and 20 mol% phosphatidylcholine. Thus, this formulation contained the same cholesterol concentration but approximately twice as much sphingomyelin as AVE95 liposomes. A reduced antitumor activity was observed for liposomes containing 30 mol% sphingomyelin compared to AVE95 containing only 14 mol% sphingomyelin demonstrating that AVE95 are superior over the high SM-concentration type of liposomes (Fig. 7).

**Example 13: Encapsulation of mitoxantrone into AVE9 liposomes**

Mitoxantrone (MXR) was encapsulated into AVE9 or AVE95 liposomes (see examples 1 and 7) by remote loading (Mayer et al. (1986) Chem. Phys. Lipids 40:333-345). For this purpose lipid films were rehydrated with 300 mM citrate buffer pH 4.0. After extrusion the pH of the external aqueous solution was adjusted to pH 7.4 with 2 N NaOH. Liposomes were heated to 55°C and mitoxantrone in PBS was added and incubated for 15 min. A drug to lipid ratio of 1:10 (w/w) was routinely used in these experiments. Unencapsulated mitoxantrone was removed by ultrafiltration using Vivaspin columns. Encapsulation efficiency was determined by reverse-phase HPLC analysis. By this approach approximately 75-85% of mitoxantrone could be routinely encapsulated. No differences in encapsulation efficiency were observed for AVE9 and AVE95. AVE95-MXR were stable upon storage at 8°C with only marginal drug leakage over a period of 4 weeks.

**Example 14: Pharmacokinetics AVE95-encapsulated mitoxantrone**

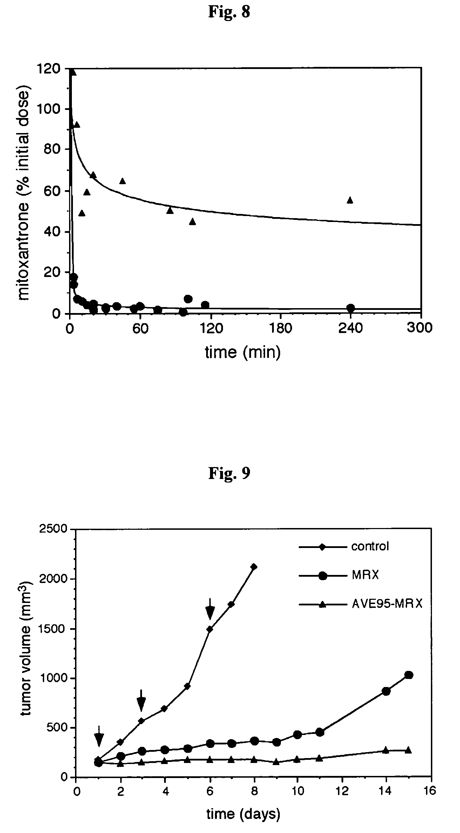

AVE95-MXR liposomes were injected i.v. into the tail veins of nude mice and blood concentration of mitoxantrone was determined by HPLC at varying time points. In this experiment we included unencapsulated mitoxantrone. AVE95-MXR caused a prolonged serum residence time of mitoxantrone (50% value = 110 min) compared to unencapsulated mitoxantrone which was rapidly cleared from circulation (50% value = < 3 min) (Fig. 8). Compared to free mitoxantrone AVE95-MXR showed a reduced accumulation of the drug in the lung, heart and kidneys 6 hours post-injection. Mitoxantrone concentrations in the liver and spleen were similar to free mitoxantrone.

**Example 15: Pharmacodynamics AVE95-encapsulated mitoxantrone**

Efficacy of AVE95-MRX was analyzed in a C26 murine colon carcinoma model. For this purpose tumor cells were injected subcutaneously into nude mice. After tumors had reached a size of approximately 50-100 mm$^3$, tumor-bearing mice were treated with mitoxantrone-loaded liposomes injecting a mitoxantrone dose of 1.5 mg/ml or 4 mg/kg body weight at days 0, 2 and 5. Unencapsulated mitoxantrone was included in these experiments. At a mitoxantrone dose of 4 mg/kg a strong inhibition of tumor growth was observed for both free and encapsulated mitoxantrone. However, at this dose tumor growth was further reduced for AVE95-encapsulated mitoxantrone compared to free mitoxantrone (Fig. 9). This improved efficacy compared to free mitoxantrone was also observed at a mitoxantrone concentration of 1.5 mg/ml.

**Claims**

1. A liposome, wherein cholesterol (CH) and sphingomyelin (SM) are present in relation to the total molar lipid composition of the liposome at a molar ratio of 30 to 60 mol% and 5 to 20 mol%, respectively.

2. The liposome of claim 1, wherein SM is present in relation to the total molar lipid composition of the liposome at a molar ratio of 10 to 18 mol%, in particular 12 to 16 mol%.

3. The liposome of claim 1 or 2, wherein CH is present in relation to the total molar lipid composition of the liposome at a molar ratio of 40 to 56 mol%, in particular 48 to 52 mol%.

4. The liposome of one of claims 1 to 3, wherein the remaining lipid of the liposome is selected from the group consisting of glycerides, glycerophospholipides, glycerophosphinolipids, glycerophosphonolipids, sulfolipids, sphingolipids, phospholipids, isoprenolides, steroids, stearines, sterols and carbohydrate containing lipids.

5. The liposome of claim 4, wherein the phospholipid is selected from the group consisting of phosphatidylcholine (PC), phosphatidylserine (PS), and phosphatidylethanolamine (PE).

6. The liposome of claim 5, wherein the PE is present in relation to the total molar lipid composition of the liposome at a molar ratio of 5 to 25 mol%.

7. The liposome of claim 5 or 6, wherein the PC is present in relation to the total molar lipid composition of the liposome at a molar ratio of 15 to 40 mol%.

8. The liposome of one of claims 1 to 7, wherein the liposome has a diameter of between 50 and 200 nm, preferably between 80 and 150 nm.

9. The liposome of one of claims 1 to 8, wherein the SM is selected from the group consisting of SM derived from milk, SM derived from egg yolk, SM derived from brain, and synthetic SM.

10. The liposome of one of claims 1 to 9, wherein the PE is selected from the group consisting of PE derived from egg; PE derived from heart; PE derived from liver; PE derived from plant; PE derived from bacteria; and synthetic PE, in particular 1,2-diacyl-sn-glycero-3-PE, 1-acyl-2-acyl-sn-glycero-3-PE or 1,2-dilauroyl-sn-glycero-3-PE (DLPE).

11. The liposome of one of claims 1 to 10, wherein a targeting moiety is attached to the liposome.

12. The liposome of claim 11, wherein the targeting moiety is selected from the group consisting of a peptide or protein, in particular an antibody or fragment thereof, a single-chain antibody or fragment thereof, a receptor ligand or fragment thereof; a carbohydrate; and a ligand.

13. The liposome of one of claim 11, in which the targeting moiety is selected from the group consisting of natural or synthetic receptor-binding peptides, in particular integrin-binding peptides such as RGD-comprising peptides; growth factors, in particular VEGF, EGF, PDGF, TGFα, TGFβ, KGF, SDGF, FGF, IGF, HGF, NGF, BDNF, neurotrophine, BMF, bombesin, M-CSF, GM-CSF, thrombopoietin, erythropoietin, SCF, SDGF, oncostatin, PDEGF, endothelin; cytokines, in particular IL-1, IL-2, IL-3, IL-4, IL-5, IL-6, IL-7, IL-8, IL-9, IL-10, IL-12, IL-13, IL-14, IL-15, interferon α, β or γ, tumor necrosis factors such as TNFα, TNFβ; chemokines, in particular RANTES, MCAF, MIP-1α or β, NAP, β-thromboglobulin; peptide hormones such as SRH, SIH, STH, MRH, MSH, PRH, PIH, prolactin, LH-RH, FSH-RH, LH/ICSH, FSH, TRH, TSH, CRH, ACTH, agiotensin, kinine, histamine; steroid hormones, in particular estrogene, gestagene, androgene, glucocorticoide; mineral corticoids; or homologous or analogous thereof; vitamins, in particular folic acid; adhesion molecules, in particular lewis X, S-lewis X, LFA-1, MAC-1, VLA-4, PECAM, vitronectin, GMP-140, ICAM-1, VCAM-1, fibronectin, laminin, B7, CD28, CD40, CD40L and selectins; viral coatproteins; monosaccharides, in particular glucose, mannose; and oligosaccharides, in particular Man2, Man3, Man4, Man5, Man6, Man7, Man8, or Man9, lewis Y, sialyl lewis Y, and lectines.

14. The liposome of one of claims 11 to 13, wherein the targeting moiety is attached to a spacer.

15. The liposome of claim 14, wherein the spacer has a length of between 1 and 10 nm, preferably between 2.5 and 5 nm.

16. The liposome of one of claims 11 to 15, wherein the targeting moiety is attached to a lipid.

17. The liposome of claim 16, wherein the lipid is selected from the group consisting of N-caproylamine-PE, N-dodecanylamine-PE, phosphatidylthioethanol, N-[4-(p-maleimidomethyl)cyclohexane-carboxamide-PE (N-MCC-PE), N-[4-(p-maleimidophenyl)butyramide]-PE (N-MPB-PE), N-[3-(2-pyridyldithio)propionate]-PE (N-PDP-PE), N-succinyl-PE, N-glutaryl-PE, N-dodecanyl-PE, N-biotinyl-PE, N-biotinyl-Cap-PE, phosphatidyl-(ethylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoyl-glycerosuccinyl-lysine. alpha-methoxy-omega-(1,2-dioctadecenoyloxy glyceryl) (DO), and alpha-methoxy-omega-(1,2-ditetradecenoyloxy glyceryl) (DT).

18. The liposome of one of claims 1 to 17, wherein one or more drugs and/or diagnostics are comprised in the liposome.

19. The liposome of claim 18, wherein the drug is selected from the group consisting of analgetics, antirheumatics, anthelminthics, antiallergics, antianemics, antiarrhythmics, antibiotics, antiinfectives, antidemenics (nootropics), antidiabetics, antidotes, antiemetics, antivertiginosics,, antiepileptics, antihemorrhagics, antihypertonics, antihypotonics, anticoagulants, antimycotics, antitussiv agents, antiviral agents, beta-receptor and calcium channel antagonists, broncholytic and antiastmatic agents, chemokines, cytokines, mitogens, cytostatics, cytotoxic agents and prodrugs thereof, dermatics, hypnotics and sedatives, immunosuppressants, immunostimulants, peptide or protein drugs, in particular hormones and physiological or pharmacological inhibitors of mitogens, chemokines, or cytokines or their respective prodrugs.

20. The liposome of claim 19, wherein the cytostatics and cytotoxic drugs are selected from the group consisting of alkylating substances, anti-metabolites, antibiotics, epothilones, nuclear receptor agonists and antagonists, anti-androgenes, anti-estrogens, platinum compounds, hormones and antihormones, interferons and inhibitors of cell cycle-dependent protein kinases (CDKs), inhibitors of cyclooxygenases and/or lipoxygenases, biogeneic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon $\alpha$, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof.

21. The liposome of claim 18, wherein the diagnostic is selected from the group consisting of an electron dense molecule, a paramagnetic molecule, a superparamagnetic molecule, a radioactive molecule, a non-radioaktive isotope, and a fluorescent molecule.

22. The liposome of one of claims 1 to 21, wherein stabilizers, protectants, metal ion chelators, buffers and/or additives are comprised in the liposome.

23. The liposome of one of claims 1 to 22, which is dried, preferably freeze dried.

24. A method for producing a liposome of one of claims 1 to 23, in which SM, CH and remaining lipid(s) are mixed.

25. The method according to claim 24 in which the remaining lipid is selected from PE and PC.

26. A pharmaceutical composition comprising a liposome of one of claims 1 to 23 or produced according to the method of claim 24, further comprising stabilizers, protectants, metal ion chelators, buffers and/or additives.

27. A diagnostic composition comprising a liposome of one of claims 1 to 23 or produced according to the method of claim 24, further comprising stabilizers, protectants, metal ion chelators, buffers and/or additives.

28. The liposome of claim 22 or 23, the pharmaceutical composition of claim 25 or the diagnostic composition of claim 27, wherein the stabilizers are selected from the group consisting of $\alpha$-tocopherol, vitamin E or carbohydrates, in particular glucose, sorbitol, sucrose, maltose, trehalose, lactose, cellubiose, raffinose, maltotriose, or dextran.

29. Use of a liposome of one of claims 1 to 23 or a pharmaceutical composition of claim 26 or 28 for the production of a medicament for the therapy of proliferative diseases, autoimmune diseases, infectious diseases, cardiovascular diseases, rheumatoid diseases, inflammatory diseases or any disease or condition, which is associated with damage to or a permeability increase of the vasculature or the activation of endothelial cells.

30. The use of claim 29, wherein the proliferative disease is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

31. Use of a diagnostic composition of claim 27 for the diagnosis of a disease selected from the group of proliferative diseases, autoimmune diseases, infectious diseases, cardiovascular diseases, rheumatoid diseases, and inflammatory diseases.

**Fig. 1**

**Fig. 2**

**Fig. 3**

A549    MSM    B254    C6    MeWo

0 - cells alone

1 - AVE9

2 - AVE3

**Fig. 4**

**Fig. 5**

**Fig. 6**

**Fig. 7**

**Fig. 8**

**Fig. 9**

**European Patent Office**

**PARTIAL EUROPEAN SEARCH REPORT**
which under Rule 45 of the European Patent Convention shall be considered, for the purposes of subsequent proceedings, as the European search report

Application Number
EP 03 02 7944

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 5 741 516 A (WEBB MURRAY S ET AL) 21 April 1998 (1998-04-21) * column 2, line 35 - line 37 * --- | 1-31 | A61K9/127 A61K47/00 A61K38/00 A61K49/22 |
| A | ARMENGOL X ET AL: "PHYSICAL STABILITY OF DIFFERENT LIPOSOME COMPOSITIONS OBTAINED BY EXTRUSION METHOD" JOURNAL OF MICROENCAPSULATION, TAYLOR AND FRANCIS INC. LONDON, GB, vol. 12, no. 5, 1 September 1995 (1995-09-01), pages 525-535, XP000529054 ISSN: 0265-2048 * abstract; table 3 * ----- | 1 | |

**TECHNICAL FIELDS SEARCHED (Int.Cl.7)**

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 31 is directed to a diagnostic method practised on the human/animal body (Article 52(4) EPC), the search has been carried out and based on the alleged effects of the compound/composition.

| Place of search | Date of completion of the search | Examiner |
|---|---|---|
| MUNICH | 10 February 2004 | Nichogiannopoulou, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C07)

**ANNEX TO THE EUROPEAN SEARCH REPORT
ON EUROPEAN PATENT APPLICATION NO.**

EP 03 02 7944

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2004

| Patent document cited in search report | Publication date | Patent family member(s) | Publication date |
|---|---|---|---|
| US 5741516      A | 21-04-1998 | US   5543152  A | 06-08-1996 |
| | | US   5814335  A | 29-09-1998 |
| | | AT    248586  T | 15-09-2003 |
| | | AU   2709495  A | 15-01-1996 |
| | | CA   2193502  A1 | 28-12-1995 |
| | | WO   9535094  A1 | 28-12-1995 |
| | | DE  69531701  D1 | 09-10-2003 |
| | | EP    0804159  A1 | 05-11-1997 |
| | | JP   3270478  B2 | 02-04-2002 |
| | | JP  10501534  T | 10-02-1998 |

EPO FORM P0459

For more details about this annex : see Official Journal of the European Patent Office, No. 12/82